# FASCICULE DE BREVET EUROPEEN

(11) **EP 0 555 467 B1**
(45) Date de publication et mention de la délivrance du brevet: **02.08.1995**
(21) Numéro de dépôt: 92919421.5
(22) Date de dépôt: 28.08.1992
(51) Int. Cl.: A61K 7/48, A61K 7/00, A61K 7/02, A61K 7/035, C09C 3/00, C09C 1/30

(54) **MATIERE EN PARTICULES COMPRENANT DES PARTICULES REVETUES DE NITRURE DE BORE, UTILE DANS DES COMPOSITIONS COSMETIQUES ET PROCEDE POUR SA PREPARATION**
TEILCHENFÖRMIGES MATERIAL, DAS AUS MIT BORNITRID ÜBERZOGENEN PARTIKELN BESTEHT, ZUR VERWENDUNG IN KOSMETISCHEN ZUSAMMENSETZUNGEN UND VERFAHREN ZUR HERSTELLUNG DIESES MATERIALS
PARTICULATE MATERIAL COMPRISING BORON NITRIDE COATED PARTICULES, FOR USE IN COSMETIC COMPOSITIONS AND PROCESS FOR THE PREPARATION OF SAID MATERIAL

(30) Priorité: 29.08.1991 US 751701
(43) Date de publication de la demande: 18.08.1993
(73) Titulaire: L'OREAL, 75008 Paris (FR)
(72) Inventeur: FARER, Alan, M., Morganville, NJ 07751 (US); BURDZY, Elisa, L., Clifton, NJ 07013 (US); HANNA, Fifi, Kearny, NJ 07032 (US); PENICNAK, A., John, Mountain Lakes, NJ 07046 (US)
(74) Mandataire: Stalla-Bourdillon, Bernard
(86) Numéro de dépôt international: FR9200830
(87) Numéro de publication internationale: WO9304668

(56) Documents cités:
- EP-A- 0 254 612
- EP-A- 0 447 287
- EP-A- 0 486 394
- US-A- 4 988 503
- US-A- 5 030 446
- US-A- 5 106 838
- PATENT ABSTRACTS OF JAPAN vol. 12, no. 28 (C-471)
- PATENT ABSTRACTS OF JAPAN vol. 10, no. 280 (C-374)(2336)

## Description

L'invention concerne une matière en particules pour cosmétiques comprenant des particules substrat, un agent de couplage silane ou titanate fixé par covalence à la surface extérieure des particules substrat et un revêtement fixé par covalence à l'agent de couplage. L'invention concerne également des compositions cosmétiques contenant cette matière en particules, ainsi qu'un procédé pour leur préparation.

On utilise depuis très longtemps des préparations cosmétiques pour améliorer l'aspect de la peau et des cheveux. Bien que la plupart des produits cosmétiques aient des compositions relativement simples, ils contiennent souvent des ingrédients qui sont incompatibles, difficiles à mettre en oeuvre et qui n'adhèrent pas à la peau. Ces problèmes se posent particulièrement dans le cas des compositions telles que les fards à paupières et les fards à joues en plaquettes qui contiennent des particules et nécessitent une préparation soigneuse pour l'obtention d'une poudre compressible adhérant bien à la peau.

Pour améliorer la dispersion et les caractéristiques apparentées, on a revêtu des constituants cosmétiques de titanates (US 4 877 604).

La présente invention comprend l'emploi de nitrure de bore comme substance complémentaire de revêtement pour les matières en particules.

Le nitrure de bore est bien connu dans le domaine des cosmétiques et peut être ajouté sous une forme libre à des cosmétiques en poudre pour assurer la douceur, la stabilité, l'adhésivité et la durabilité (demandes de brevets japonais 61-28596 et 62-49247).

Cependant, le nitrure de bore est une matière coûteuse et très dense, et il serait avantageux de diminuer les quantités de nitrure de bore, tout en assurant les mêmes améliorations des propriétés tactiles de la préparation cosmétique finie et de réduire ainsi la densité et le coût.

On a découvert que le revêtement d'une particule avec du nitrure de bore, en utilisant un agent de couplage silane ou titanate, facilite la mise en oeuvre, réduit le coût et améliore les propriétés tactiles.

La présente invention fournit donc une matière en particules pour cosmétiques, comprenant des particules substrat, au moins un agent de couplage silane ou titanate, fixé par covalence à la surface extérieure des particules substrat, et un revêtement fixé par covalence à l'agent de couplage, le revêtement étant caractérisé en ce qu'il est fait de nitrure de bore.

Lorsqu'on l'incorpore à des compositions cosmétiques, la matière en particules de l'invention assure des propriétés esthétiques exceptionnelles à des préparations telles que des produits en poudre, des mascaras, des émulsions (y compris des produits de maquillage et des produits hydratants), des rouges à lèvres, des eye-liners, des contours de lèvres, des fards à joues en crème, des vernis à ongles, des produits de maquillage en crème et des démaquillants contenant éventuellement un agent nettoyant, de type "scrub".

De plus, l'emploi de particules substrat revêtues de nitrure de bore dans des compositions cosmétiques accroît leur fluidité et améliore la dispersion.

Les particules substrat revêtues de nitrure de bore de l'invention ont une granulométrie moyenne dans la gamme de 5 à 550 »m, de préférence de 5 à 250 »m et tout préférablement de 5 à 100 »m.

Les particules substrat non revêtues peuvent être sous forme de particules irrégulières ou de sphères ayant une granulométrie moyenne de 2 à 500 »m, de préférence de 5 à 200 »m, tout préférablement de 5 à 80 »m.

Le nitrure de bore utilisé selon l'invention peut avoir une granulométrie moyenne de 0,1 à 40 »m, de préférence de 1 à 15 »m.

La granulométrie moyenne du nitrure de bore peut donc être supérieure à celle des particules substrat. Cependant, on préfère que le rapport du diamètre moyen des particules de nitrure de bore au diamètre moyen des particules substrat varie d'environ 0,02 à 20, mieux de 0,05 à 8. Des valeurs élevées de ce rapport des tailles sont possibles par suite du caractère non sphérique de certaines particules substrat et également du nitrure de bore.

Selon l'invention, le nitrure de bore est présent à raison de 15 à 99 %, mieux de 50 % à 99 % et de préférence de 80 à 97 % du poids des particules revêtues. L'étendue de cette gamme résulte de la densité très variable des particules substrat.

La forme tout particulièrement préférée du nitrure de bore utilisé pour revêtir les particules selon l'invention est le nitrure de bore hexagonal. Une gamme de produits appropriés est constituée des poudres de nitrure de bore Combat® de Standard Oil Engineered Materials Company, Niagara Falls, New York, USA ; on préfère les formes très pures, en particulier la qualité SHP325.

L'agent de couplage silane et/ou titanate convenant à l'emploi dans l'invention est, dans le premier cas, de préférence un trialcoxysilane et, dans le second cas, de préférence un monoalkyltitanate (souvent appelé également monoalcoxytitanate) ou un produit de coordination phospho-titanate.

Les trialcoxysilanes (notamment triméthoxy et triéthoxysilanes) utilisables sont en particulier les alkyltrialcoxysilanes et les perfluoroalkyltrialcoxysilanes dont les groupements alkyle ont notamment 1 à 20 atomes de carbone, par exemple l'isobutyltriméthoxysilane.

Des monoalkyl titanates utiles comprennent les monoalkyl triisostéaroyl titanates, les monoalkyl diisostéaroyl méthacryloyl titanates, les monoalkyl isostéaroyl diméthacryloyl titanates et les monoalkyl triméthacryloyl titanates, dont le groupe alkyle a par exemple de 1 à 20 atomes de carbone.

On utilise de préférence l'isopropyl isostéaroyl diméthacryloyl titanate ou l'isopropyl triisostéaroyl titanate, ce dernier étant tout particulièrement préféré.

Des produits de coordination phospho-titanates comprennent le di(dioctyl)phosphitotitanate de tétra-isopropyle et le di(ditridécyl) phosphitotitanate de tétra(2,2-diallyloxyméthyl)butyle.

L'agent de couplage est normalement liquide à la température ordinaire et peut être appliqué tel quel aux particules substrat. Sinon, l'agent de couplage peut être dissous ou dilué dans l'eau ou dans un solvant organique, tel que le benzène, le toluène ou la butane-2-one, avant l'emploi.

Les particules substrat peuvent être faites d'un polymère naturel ou synthétique ou d'une matière minérale (amorphe ou cristalline), y compris le polypropylène, les polycarbonates, la poudre de silicone, la cellulose, les résinesurée-formaldéhyde, la gélatine réticulée, le collagène, la kératine, la silice, l'alumine, le dioxyde de titane, l'oxyde de zinc, l'oxyde de zirconium, le silicate de calcium, les perles de verre, les stéarates et les myristates métalliques, tels que le stéarate de zinc et le myristate de magnésium, le mica et les copolymères de polyvinylidène. De préférence, les particules substrat sont faites de nylon, d'amidon, de polyméthacrylate de méthyle, de polyéthylène basse densité, de silice, d'un copolymère de polyvinylidène ou de polystyrène.

Les particules substrat préférées sont sous forme de microsphères faites d'une matière thermoplastique non toxique et non irritante. Des matières appropriées comprennent les polymères ou copolymères de dérivés d'éthylène, le polyéthylène, le polystyrène, les copolymères de chlorure de vinyle/acrylonitrile, les polyesters, les polyamides, les polymères d'urée-formaldéhyde et les copolymères de chlorure de vinylidène, tels que les copolymères de chlorure de vinylidène/acrylonitrile, et similaires.

On préfère tout particulièrement des microsphères creuses de copolymère de polyvinylidène ayant une granulométrie moyenne de 5 à 40 »m et une masse volumique de 0,01 à 0,1, de préférence de 0,01 à 0,065 g/cm³. La partie creuse des microsphères peut être remplie d'un gaz, typiquement d'un hydrocarbure, de préférence l'isobutane. Les microsphères creuses peuvent être préparées selon des procédés connus, tels que deux décrits dans le brevet US 3 615 972 et la demande de brevet européen 056 219. Des microsphères appropriées de copolymère de chlorure de vinylidène/acrylonitrile sont fournies par la Société Kemanord Plast sous le nom commercial d'Expancel 551 DE 20 (granulométrie moyenne : 25 »m) et d'Expancel 551 DE (granulométrie moyenne : 40 »m). La granulométrie moyenne est mesurée par diffraction laser avec un Malvern Mastersizer®.

Les microsphères creuses de polyvinylidène de ce mode de réalisation ont une masse volumique extrêmement faible et sont normalement difficiles à incorporer à une poudre cosmétique compressible. Par revêtement de ces microsphères avec un agent de couplage, puis avec du nitrure de bore selon l'invention, on obtient une matière de faible densité qu'il est facile de disperser dans des compositions cosmétiques pour obtenir un produit qui est compressible, qui adhère à la peau et qui possède d'excellentes propriétés tactiles. On a découvert que, de façon surprenante, les compositions cosmétiques contenant des microsphères de copolymère de polyvinylidène revêtues de nitrure de bore ont des propriétés tactiles et autres qui sont supérieures à celles des compositions cosmétiques dans lesquelles le nitrure de bore et les microsphères de copolymère de polyvinylidène sont incorporés sous forme de composants séparés. Les compositions contenant des microsphères creuses revêtues de nitrure de bore sont plus denses, adhèrent mieux à la peau et présentent une meilleure compressibilité. De plus, les microsphères revêtues de nitrure de bore peuvent être utilisées à des taux plus élevés sans difficultés de mise en oeuvre.

La présente invention concerne également des compositions cosmétiques contenant la matière en particules exposée ci-dessus, de préférence en une proportion de 0,2 à 90 et mieux de 0,5 a 50 % en poids du poids de la composition.

Ces compositions peuvent être présentées sous l'une quelconque des formes cosmétiques indiquées ci-dessus et peuvent contenir des ingrédients classiques utilisés dans les cosmétiques, tels que des pigments, des charges, des huiles, des cires, des liants, des humectants, des parfums, des conservateurs, etc.

Les compositions cosmétiques peuvent également contenir une matière en particules revêtues, telle que du talc revêtu de l'ester trimyristique du glycérol (appelé "trimyristine").

Une charge utile pour le mélange sous forme d'une poudre aux compositions selon l'invention est le polytétrafluoroéthylène vendu aux E.U.A. par Hoechst-Celanese Corp. sous le nom de "Ceridust 9205F".

La présente invention concerne également un procédé pour la préparation de la matière en particules précédemment définie. Ce procédé est caractérisé en ce que l'on traite tout d'abord les particules substrat avec 0,01 à 5,0 % en poids des particules substrat d'un agent de couplage silane ou titanate, avec agitation continue, pour former un mélange homogène auquel on ajoute une suspension fluide du nitrure de bore et, après nouvelle agitation, on sépare les particules revêtues.

Dans ce procédé, la suspension fluide de nitrure de bore est de préférence une suspension aqueuse et l'agent de couplage est de préférence ajouté à la suspension aqueuse des particules substrat non revêtues. On sépare les particules revêtues, par exemple par filtration.

Un mode de mise en pratique de ce procédé consiste à ajouter environ 2 % (du poids des particules substrat) d'un agent de couplage liquide à une dispersion, ou suspension, aqueuse uniforme des particules substrat, à la température ordinaire, dans des conditions d'agitation rapide. Après 30 à 60 minutes d'agitation, on ajoute une suspension aqueuse de nitrure de bore au mélange en poursuivant l'agitation rapide. On agite le mélange pendant encore 20 à 60 minutes, puis on sépare par filtration les particules revêtues. On peut désagréger le résidu par passage à travers un tamis grossier.

Un autre mode de réalisation du procédé selon l'invention comprend tout d'abord la pulvérisation de l'agent de couplage liquide sur un lit fluidisé ou agité des particules substrat, puis la dispersion dans l'eau des particules traitées avec l'agent de couplage, dans des conditions de mélange rapide et à la température ordinaire, pour former une suspension ou mélange aqueux. On ajoute ensuite au mélange une suspension de nitrure de bore et on agite rapidement à la température ordinaire pendant 20 à 60 minutes. Puis, par filtration, on sépare les particules revêtues du mélange.

Les exemples suivants sont présentés pour illustrer l'invention et ne doivent pas être conçus comme en limitant la portée.

### EXEMPLE 1 :

### Préparation de particules de copolymère de polyvinylidène revêtues de nitrure de bore

On ajoute 2 g d'isopropyl triisostéaroyltitanate (ITT) à une dispersion de 20 g d'Expancel 551 DE 20 dans l'eau, en utilisant un mélangeur de type Lightnin. On effectue l'addition du titanate à la température ordinaire en agitant rapidement. On poursuit l'agitation après l'addition de l'agent de couplage titanate pendant 60 minutes. On ajoute ensuite au mélange, en agitant rapidement à la température ordinaire, une suspension aqueuse de 78 g de poudre de nitrure de bore (BN) Grade SHP325, ayant une granulométrie de 30 à 40 »m. On agite le mélange obtenu pendant 60 minutes. On sépare ensuite les particules revêtues d'avec le mélange par filtration à travers un papier-filtre n° 1. On lave ensuite les particules revêtues avec de l'eau alors qu'elles sont encore sur le filtre, puis on sèche. On désagrège ensuite deux fois les particules revêtues séchées avec un micropulvérisateur et on fait passer à travers un tamis de 508 »m (0,020 inch) pour obtenir un produit ayant une granulométrie moyenne de 25 »m avec une granulométrie maximale de 50 »m.

### EXEMPLES 2 A 9 :

On reprend le mode opératoire de l'exemple 1 en utilisant différentes particules substrat en les quantités indiquées dans le tableau suivant.

**TABLEAU**

| Ex. | Particules substrat | Rapport granulométrique* | % p | | |
|---|---|---|---|---|---|
| | | | BN | ITT | Particules substrat |
| 2 | Sphères creuses de polyéthylène ; granulométrie moyenne : 10 »m | 3-4 | 95 | 2 | 3 |
| 3 | Amidon ; Dry Flo®, National Starch ; granulométrie moyenne : 20 »m | 1,5-2 | 33 | 2 | 65 |
| 4 | Alumine ; granulométrie moyenne : 8 »m | 4,75-5 | 63 | 2 | 35 |
| 5 | Polyméthacrylate de méthyle ; 2-15 »m | 2-20 | 33 | 2 | 65 |
| 6 | Poudre de nylon (5 »m) ; sphères creuses | 6-8 | 33 | 2 | 65 |
| 7 | Idem | 6-8 | 28 | 2 | 70 |
| 8 | Idem | 6-8 | 18 | 2 | 80 |
| 9 | Copolymère de polyvinylidène ; 5-40 »m | 0,75-8 | 83 | 2 | 15 |

| | | | | | |
|---|---|---|---|---|---|
| * rapport de la granulométrie du BN à la granulomérie des particules substrat. | | | | | |

### EXEMPLES DE COMPOSITIONS COSMETIQUES

### EXEMPLE A : poudre compacte

| | % p/p |
|---|---|
| Polytétrafluoroéthylène (Ceridust 9205F) | 0,90 |
| Mica vendu sous le nom de "Mica 217N-12" (Kobo Products, Inc.) | 15,00 |
| Stéarate de lithium | 2,00 |
| Oxyde de fer noir | 0,16 |
| Oxyde de fer jaune | 0,35 |
| Oxyde de fer rouge | 0,26 |
| Palmitate de zinc | 3,50 |
| Polyéthylène haute densité | 5,00 |
| Oxychlorure de bismuth | 1,00 |
| Particules revêtues de nitrure de bore de l'exemple 1 | 0,30 |
| Citrate de triisocétyle | 0,38 |
| Mica | 29,10 |
| Diméthicone | 7,00 |
| Phénoxyéthanol | 0,05 |
| Méthyldibromoglutaronitrile | 0,01 |
| Talc revêtu de 3 % de trimyristine | 34,99 |
| | 100,00 |

Pour préparer la poudre cosmétique compacte, on mélange tous les ingrédients sauf le liant diméthicone dans un mélangeur Baker-Perkins à une température contrôlée de 40°C pendant environ 20 minutes. On ajoute lentement le liant en une période de 15 minutes en mélangeant à 40°C. On décharge ce lot du mélangeur et on pulvérise par passage à travers un micropulvérisateur ayant un tamis de 508 »m (0,020 inch). On presse ensuite le lot en gâteaux en utilisant une pression de 1,38 kPa (2 000 psi) pour obtenir une poudre compact ayant d'excellentes caractéristiques.

### EXEMPLE B : fard à joues

| | % p/p |
|---|---|
| Mica vendu sous le nom de "Mica 217N-12" (Kobo Products, Inc.) | 15,00 |
| Oxyde de fer rouge | 2,00 |
| Oxyde de fer jaune | 0,24 |
| Oxyde de fer noir | 0,40 |
| Laque d'aluminium D&C red#30 | 0,75 |
| Bleu d'outremer | 0,58 |
| Particules revêtues de nitrure de bore de l'exemple 1 | 0,30 |
| Mica traité avec un titanate | 10,00 |
| Diméthicone | 8,90 |
| Méthyldibromoglutaronitrile/phénoxyéthanol | 0,07 |
| Polytétrafluoroéthylène (Ceridust 9205F) | 8,27 |
| Stéarate de zinc | 3,00 |
| Parfum | 0,15 |
| Mica ; Sericite 281® ; Whittaker, Clark & Daniels | 50,34 |
| | 1̅0̅0̅,̅0̅0̅ |

On prépare ce fard à joues en poudre par mélange de tous les ingrédients, sauf le liant diméthicone, dans un mélangeur Baker-Perkins à une température contrôlée de 40°C pendant environ 20 minutes. On décharge ensuite le lot du mélangeur et on pulvérise par passage à travers un micropulvérisateur ayant un tamis de 508 »m (0,020 inch). On replace le lot dans le mélangeur Baker-Perkins, puis on ajoute lentement le liant en une période de 15 minutes en mélangeant à 40°C. Lorsque le mélange est achevé, on décharge le produit du mélangeur, on fait passer à nouveau à travers le micropulvérisateur ayant un tamis de 508 »m (0,020 inch), puis on comprime en un gâteau avec une pression de 2,76 kPa (4 000 psi) pour obtenir un produit ayant d'excellentes caractéristiques.

## Revendications

1. Matière en particules pour produits cosmétiques comprenant des particules substrat, un agent de couplage silane ou titanate fixé par covalence à la surface extérieure des particules substrat et un revêtement fixé par covalence à l'agent de couplage, caractérisée en ce que le revêtement est du nitrure de bore.

2. Matière en particules selon la revendication 1 ou 2, caractérisée en ce que les particules substrat sont faites d'une matière polymère ou minérale.

3. Matière en particules selon la revendication 1 ou 2, caractérisée en ce que les particules substrat sont polymères, éventuellement creuses et sont faites d'une matière choisie parmi le nylon, l'amidon, le polyméthacrylate de méthyle, le polyéthylène basse densité, un copolymère de polyvinylidène et le polystyrène.

4. Matière en particules selon l'une quelconque des revendications précédentes, caractérisée en ce que les particules substrat sont faites d'un copolymère de chlorure de vinylidène/acrylonitrile et sont sous forme de microsphères creuses.

5. Matière en particules selon la revendication 4, caractérisée en ce que les microsphères creuses sont remplies d'un gaz inerte, de préférence l'isobutane, et ont une masse volumique entre 0,01 et 0,1 g/cm³.

6. Matière en particules selon la revendication 1 ou 2, caractérisée en ce que les particules substrat sont faites d'une matière minérale qui est la silice ou l'alumine.

7. Matière en particules selon l'une quelconque des revendications précédentes, caractérisée en ce que le rapport de la granulométrie des particules de nitrure de bore à la granulométrie des particules substrat est de 0,02 à 20, de préférence de 0,05 à 8.

8. Matière en particules selon l'une quelconque des revendications précédentes, caractérisée en ce que l'agent de couplage est un monoalcoxytitanate ou un produit de coordination phosphotitanate.

9. Matière en particules selon l'une quelconque des revendications précédentes, caractérisée en ce que l'agent de couplage est choisi par'si les monoalkyl triisostéaroyl titanates, les monoalkyl diisostéaroyl méthacryloyl titanates, les monoalkyl diméthacryloyl isostéaroyl titanates et les monoalkyl triméthacryloyl titanates dont le groupe alcoxy a de 1 à 20 atomes de carbone.

10. Matière en particules selon l'une quelconque des revendications précédentes, caractérisée en ce que l'agent de couplage est l'isopropyl triisostéaroyltitanate.

11. Matière en particules selon l'une quelconque des revendications précédentes, caractérisée en ce que les particules substrat ont une granulométrie de 2 à 500 »m, de préférence de 5 à 200 »m, tout préférablement de 5 à 80 »m.

12. Matière en particules selon l'une quelconque des revendications précédentes, caractérisée en ce que les particules substrat revêtues ont une granulométrie de 5 à 550 »m, de préférence de 5 à 250 »m, tout préférablement de 5 à 100 »m.

13. Matière en particules selon l'une quelconque des revendications précédentes, caractérisée en ce que le nitrure de bore est hexagonal et a une granulométrie de 0,1 à 40 »m, de préférence de 1 à 15 »m.

14. Matière en particules selon l'une quelconque des revendications précédentes, caractérisée en ce que le nitrure de bore est présent à raison de 15 à 99 %, de préférence de 50 à 99 %, du poids des particules revêtues.

15. Composition cosmétique caractérisée en ce qu'elle contient une matière en particules selon l'une quelconque des revendications 1 à 14.

16. Composition cosmétique selon la revendication 15, caractérisée en ce qu'elle comprend de 0,2 à 90 et de préférence de 0,5 à 50 % en poids de la matière en particules.

17. Composition cosmétique selon la revendication 15 ou 16, caractérisée en ce qu'elle contient aussi des ingrédients cosmétiques classiques, tels que des pigments, des charges, des huiles, des cires, des liants, des humectants, des conservateurs et des parfums.

18. Procédé pour la préparation de la matière en particules de l'une quelconque des revendications 1 à 14, caractérisé en ce que l'on traite tout d'abord les particules substrat avec 0,01 à 50 % en poids, relativement au poids des particules substrat, d'un agent de couplage silane ou titanate, avec agitation continue pour former un mélange homogène, auquel on ajoute une suspension fluide de nitrure de bore et, après poursuite de l'agitation, on sépare les particules revêtues.

19. Procédé selon la revendication 18, caractérisé en ce que la suspension fluide de nitrure de bore est une suspension aqueuse.

20. Procédé selon les revendications 18 et 19, caractérisé en ce que l'agent de couplage est pulvérisé sur un lit agité des particules substrat.

## Claims

1. Material in the form of particles for cosmetic products comprising substrate particles, a silane or titanate coupling agent bound covalently to the outer surface of the substrate particles and a coating bound covalently to the coupling agent, characterized in that the coating is boron nitride.

2. Material in the form of particles according to Claim 1, characterized in that the substrate particles are made of a polymeric or inorganic material.

3. Material in the form of particles according to Claim 1 or 2, characterized in that the substrate particles are polymeric, optionally hollow and are made of a material chosen from nylon, starch, polymethyl methacrylate, low-density polyethylene, a polyvinylidene copolymer and polystyrene.

4. Material in the form of particles according to any one of the preceding claims, characterized in that the substrate particles are made of a vinylidene chloride/acrylonitrile copolymer and are in the form of hollow microspheres.

5. Material in the form of particles according to Claim 4, characterized in that the hollow microspheres are filled with an inert gas, preferably isobutane, and have a density between 0.01 and 0.1 g/cm³.

6. Material in the form of particles according to Claim 1 or 2, characterized in that the substrate particles are made of an inorganic material which is silica or alumina.

7. Material in the form of particles according to any one of the preceding claims, characterized in that the ratio of the particle size of the boron nitride particles to the particle size of the substrate particles is from 0.02 to 20, preferably from 0.05 to 8.

8. Material in the form of particles according to any one of the preceding claims, characterized in that the coupling agent is a monoalkoxytitanate or a phosphotitanate coordination product.

9. Material in the form of particles according to any one of the preceding claims, characterized in that the coupling agent is chosen from monoalkyl triisostearoyltitanates, monoalkyl diisostearoylmethacryloyltitanates, monoalkyl dimethacryloylisostearoyltitanates and monoalkyl trimethacryloyltitanates in which the alkoxy group has from 1 to 20 carbon atoms.

10. Material in the form of particles according to any one of the preceding claims, characterized in that the coupling agent is isopropyl triisostearoyltitanate.

11. Material in the form of particles according to any one of the preceding claims, characterized in that the substrate particles have a particle size of 2 to 500 »m, preferably of 5 to 200 »m, very preferably of 5 to 80 »m.

12. Material in the form of particles according to any one of the preceding claims, characterized in that the coated substrate particles have a particle size of 5 to 550 »m, preferably of 5 to 250 »m, very preferably of 5 to 100 »m.

13. Material in the form of particles according to any one of the preceding claims, characterized in that the boron nitride is hexagonal and has a particle size of 0.1 to 40 »m, preferably of 1 to 15 »m.

14. Material in the form of particles according to any one of the preceding claims, characterized in that the boron nitride is present in a proportion of 15 to 99 %, preferably of 50 to 99 %, of the weight of the coated particles.

15. Cosmetic composition characterized in that it contains a material in the form of particles according to any one of Claims 1 to 14.

16. Cosmetic composition according to Claim 15, characterized in that it comprises from 0.2 to 90 and preferably from 0.5 to 50 % by weight of the material in the form of particles.

17. Cosmetic composition according to Claim 15 or 16, characterized in that it also contains conventional cosmetic ingredients such as pigments, fillers, oils, waxes, binders, moisteners, stabilizers and perfumes.

18. Process for the preparation of the material in the form of particles of any one of Claims 1 to 14, characterized in that the substrate particles are first of all treated with 0.01 to 50 % by weight, relative to the weight of the substrate particles, of a silane or titanate coupling agent, with continuous agitation to form a homogeneous mixture, to which a fluid suspension of boron nitride is added and, after continuing the agitation, the coated particles are isolated.

19. Process according to Claim 18, characterized in that the fluid suspension of boron nitride is an aqueous suspension.

20. Process according to Claims 18 and 19, characterized in that the coupling agent is sprayed onto an agitated bed of the substrate particles.

## Patentansprüche

1. Teilchenförmiges Material für kosmetische Produkte, das Substratteilchen, ein Kupplungsmittel aus Silan oder Titanat, das kovalent auf der äußeren Oberfläche der Substratteilchen gebunden ist, und einen kovalent an das Kupplungsmittel gebundenen Überzug aufweist, dadurch gekennzeichnet, daß der Überzug aus Bornitrid besteht.

2. Teilchenförmiges Material gemäß Anspruch 1 (oder 2), dadurch gekennzeichnet, daß die Substratteilchen aus einem polymeren oder mineralischen Material hergestellt sind.

3. Teilchenförmiges Material gemäß Anspruch 1 oder 2, dadurch gekennzeichnet, daß die Substratteilchen gegebenenfalls hohle Polymere darstellen und aus einem aus Nylon, Stärke, Methylpolymethacrylat, Polyethylenen niedriger Dichte, einem Polyvinylidencopolymer und Polystyrol ausgewählten Material bestehen.

4. Teilchenförmige Material gemäß einem der vorstehenden Ansprüche, dadurch gekennzeichnet, daß die Substratteilchen aus einem Vinylidenchlorid/Acrylnitril-Copolymer bestehen und in Form von Mikrohohlkügelchen vorliegen.

5. Teilchenförmiges Material gemäß Anspruch 4, dadurch gekennzeichnet, daß die Mikrohohlkügelchen mit einem Inertgas, vorzugsweise Isobutan gefüllt sind und eine Dichte zwischen 0,01 und 0,1 g/cm³ aufweisen.

6. Teilchenförmiges Material gemäß einem der Ansprüche 1 oder 2, dadurch gekennzeichnet, daß die Substratteilchen aus einem mineralischen Material aus Kieselerde oder Tonerde bestehen.

7. Teilchenförmiges Material gemäß einem der vorstehenden Ansprüche, dadurch gekennzeichnet, daß das Verhältnis der Korngröße des Bornitrids zur Korngröße der Substratteilchen von 0,02 bis 20, vorzugsweise von 0,05 bis 8 beträgt.

8. Teilchenförmiges Material gemäß einem der vorstehenden Ansprüche, dadurch gekennzeichnet, daß das Kupplungsmittel Monoalkoxytitanat oder ein Phosphotitanat-Koordinationsprodukt ist.

9. Teilchenförmiges Material gemäß einem der vorstehenden Ansprüche, dadurch gekennzeichnet, daß das Kupplungsmittel aus den Monoalkyltriisostearoyltitanaten, den Monoalkyldiisostearoylmethacryloyltitanaten, den Monoalkyldimethacryloylisostearoyltitanaten und den Monoalkyltrimethacryloyltitanaten ausgewählt ist, in denen die Alkoxygruppe 1 bis 20 Kohlenstoffatomen aufweist.

10. Teilchenförmiges Material gemäß einem der vorstehenden Ansprüche, dadurch gekennzeichnet, daß das Kupplungsmittel Isopropyltristearoyltitanat ist.

11. Teilchenförmiges Material gemäß einem der vorstehenden Ansprüche, dadurch gekennzeichnet, daß die Substratteilchen einen Korndurchmesser von 2 bis 500 »m, vorzugsweise von 5 bis 200 »m und ganz besonders bevorzugt von 5 bis 80 »m haben.

12. Teilchenförmiges Material gemäß einem der vorstehenden Ansprüche, dadurch gekennzeichnet, daß die überzogenen Substratteilchen einen Korndurchmesser von 5 bis 550 »m, vorzugsweise von 5 bis 250 »m und ganz besonders bevorzugt von 5 bis 100 »m aufweisen.

13. Teilchenförmiges Material gemäß einem der vorstehenden Ansprüche, dadurch gekennzeichnet, daß das Bornitrid in hexagonaler Form vorliegt und eine Korngröße von 0,1 bis 40 »m, vorzugsweise 1 bis 15 »m aufweist.

14. Teilchenförmiges Material gemäß einem der vorstehenden Ansprüche, dadurch gekennzeichnet, daß das Bornitrid in einer Menge von 15 bis 99 Gew.- %, vorzugsweise von 50 bis 99 Gew.-%, bezogen auf das Gewicht der überzogenen Teilchen, vorliegt.

15. Kosmetische Zubereitung, dadurch gekennzeichnet, daß sie ein teilchenförmiges Material gemäß einem der Ansprüche 1 bis 14 enthält.

16. Kosmetische Zubereitung gemäß Anspruch 15, dadurch gekennzeichnet, daß sie von 0,2 bis 90 Gew.-% und vorzugsweise von 0,5 bis 50 Gew.-% an teilchenförmigem Material enthält.

17. Kosmetische Zubereitung gemäß einem der Ansprüche 15 oder 16, dadurch gekennzeichnet, daß sie zusätzlich herkömmliche kosmetische Bestandteile wie Pigmente, Zuschläge, Öle, Wachse, Bindemittel, Feuchthaltemittel, Konservierungsstoffe und Parfüme enthält.

18. Verfahren zur Herstellung des teilchenförmigen Materials gemäß einem der Ansprüche 1 bis 14, dadurch gekennzeichnet, daß man alle zugänglichen Stellen der Substratteilchen mit 0,01 bis 50 Gew.-% eines Kupplungsmittels aus Silan oder Titanat, bezogen auf das Gewicht der Substratteilchen, unter kontinuierlichem Rühren zum Erhalt einer homogenen Mischung behandelt, wonach man eine fluide Bornitridsuspension zufügt und, nach weiterem Rühren, die überzogenen Teilchen abtrennt.

19. Verfahren gemäß Anspruch 18, dadurch gekennzeichnet, daß die fluide Bornitridsuspension eine wäßrige Suspension darstellt.

20. Verfahren gemäß den Ansprüchen 18 und 19, dadurch gekennzeichnet, daß das Kupplungsmittel auf einem Rüttelbett aus Substratteilchen pulverisiert wird.
